# FASCICULE DE BREVET EUROPEEN

(11) **EP 1 155 710 B1**
(45) Date de publication et mention de la délivrance du brevet: **26.10.2005**
(21) Numéro de dépôt: 01401239.7
(22) Date de dépôt: 15.05.2001
(51) Int. Cl.: A61N 1/05

(54) **Nécessaire de mise en place d'une sonde de stimulation d'une cavité cardiaque implantable dans le reséau coronarien**
Vorrichtung zum Implantieren von einer Herzstimulationsleitung in Koronargefässen
Device for implanting a heart stimulation lead in coronary vessels

(30) Priorité: 16.05.2000 FR 0006248; 10.10.2000 FR 0012901
(43) Date de publication de la demande: 21.11.2001
(73) Titulaire: ELA MEDICAL, 92541 Montrouge (FR)
(72) Inventeur: Ollivier, Jean-François, 91190 Villiers le Bade (FR)
(74) Mandataire: Dupuis-Latour, Dominique

(56) Documents cités:
- EP-A- 0 993 840
- WO-A-99/55412
- US-A- 4 345 606
- US-A- 6 033 414

## Description

L'invention concerne la mise en place des sondes de stimulation cardiaque destinées à être implantées dans le réseau coronarien du coeur pour permettre la stimulation d'une cavité gauche par un "dispositif médical implantable actif" tel que défini par la directive 90/385/CEE du 20 juin 1990 du Conseil des communautés européennes, et plus précisément un dispositif tel qu'un stimulateur cardiaque, défibrillateur et/ou cardioverteur, notamment un stimulateur de type "multisite".

Le EP-A-0 993 840 (ELA Médical) décrit une sonde d'un tel type, répondant à un certain nombre de critères précis concernant notamment la géométrie et la flexibilité de sa partie distale.

Une telle sonde n'est pas une sonde endocavitaire (comme le sont les sondes de stimulation des cavités droites), mais une sonde introduite dans le réseau coronarien, avec une électrode disposée face au ventricule gauche (la stimulation de l'oreillette gauche étant également possible par le choix d'un autre site de stimulation). L'accès à l'entrée du sinus coronaire se fait via l'oreillette droite.

L'introduction d'une telle sonde, opérée par des voies endocavitaires, est une intervention particulièrement délicate, compte tenu notamment du fait que la position des points de stimulation est très importante dans le cas d'un stimulateur de type "multisite", les deux points de stimulation ventricule gauche/ventricule droit devant être les plus éloignés possible pour optimiser la resynchronisation de l'ensemble des cavités cardiaques.

L'une des techniques de mise en place d'une telle sonde met en oeuvre un accessoire dit "cathéter-guide", par exemple décrit dans le WO-A-99/55412 Un cathéter-guide est un tube creux armé par un treillis métallique, et dont la surface intérieure est une surface à faible coefficient de frottement (par exemple une surface de PTFE co-extrudée ou co-moulée avec le reste de la gaine) et dont la flexibilité est étudiée de manière à présenter une rigidité en torsion suffisante pour permettre la transmission d'un mouvement de rotation d'une extrémité à l'autre, afin de pouvoir ajuster l'orientation de l'extrémité de la sonde par rapport au myocarde. Par ailleurs, le cathéter-guide est du type *"soft-tip"*, c'est-à-dire que, dans sa région terminale, sa souplesse augmente au fur et à mesure que l'on se rapproche de l'extrémité distale, ceci afin de permettre une approche atraumatique du cathéter-guide dans le réseau coronarien et franchir des obstacles tels que des valvules sans trop de difficultés et sans blesser les tissus.

Une fois posé, le cathéter-guide constitue un "tunnel" direct entre le "monde extérieur" et le sinus coronaire, que le chirurgien peut alors utiliser pour y glisser la sonde jusqu'à son emplacement définitif.

À ce stade, une difficulté importante se présente dans l'étape de retrait du cathéter-guide, manoeuvre qui doit être bien entendu opérée sans déloger la sonde ni modifier l'orientation de celle-ci.

Jusqu'à présent, au moment de son retrait le cathéter-guide est découpé à son extrémité proximale le long d'une génératrice au moyen d'un outil de type "cutter", adapté sur cette extrémité terminale et qui vient fendre à la fois l'embout-poignée du cathéter-guide et le treillis métallique formant l'armature du tube creux.

Cette technique, consistant à découper la gaine du cathéter-guide après mise en place de la sonde, présente le risque d'endommager la sonde du fait de la présence de l'outil tranchant à faible distance (de l'ordre de 1 mm) du corps de la sonde.

Pour remédier à cet inconvénient, des accessoires de mise en place d'une structure différente ont été proposés, avec par exemple une gaine non armée et pelable. Malheureusement, le prédécoupage de cette gaine sur toute sa longueur entraîne une faiblesse et une moindre rigidité du tube, avec un risque de pliure et une moins bonne transmission des efforts exercés à son extrémité proximale lors de la mise en place.

Ainsi, il n'existe aucune solution satisfaisante à ce problème de retrait du guide de sonde, étape qui reste encore extrêmement tributaire de l'habileté du chirurgien et des aléas habituels d'une intervention complexe de cette nature.

L'un des buts de l'invention est de proposer un nécessaire de mise en place d'une telle sonde coronaire, qui pallie les difficultés précitées en permettant un retrait aisé et sûr du cathéter-guide, en s'affranchissant de la plus ou moins grande habileté de l'opérateur.

Plus précisément, le nécessaire de mise en place de l'invention comprend un cathéter-guide de type en lui-même connu, c'est-à-dire comportant une lumière interne ouverte à ses deux extrémités et apte à recevoir la sonde sur toute sa longueur.

De façon caractéristique de l'invention, ce cathéter-guide est combiné à une rallonge comportant à son extrémité distale des moyens de solidarisation à l'extrémité proximale monodiamètre de la sonde ; le diamètre extérieur des moyens de solidarisation est le même que celui du reste de la rallonge ; la surface extérieure de cette rallonge est une surface à faible coefficient de frottement par rapport à la surface intérieure de la lumière du cathéter-guide ; le diamètre extérieur de cette rallonge est inférieur au diamètre intérieur de la lumière du cathéter-guide, de manière à permettre un glissement axial du cathéter-guide sur toute la longueur de la rallonge, sans déplacement axial concomitant de la sonde.

Avantageusement, le nécessaire de l'invention peut comprendre en outre les accessoires suivants :
- un mandrin long, apte à être inséré dans une lumière interne de la rallonge par une extrémité proximale de celle-ci, la longueur de ce mandrin étant supérieure à la somme des longueurs de la sonde et de la rallonge ;
- un manchon, apte à être emboîté sur l'extrémité proximale monodiamètre de la sonde, de manière à former une fiche de connexion électrique raccordable à une tête de connecteur d'un stimulateur ou défibrillateur, très avantageusement en conformité avec la norme du système de connexion IS-1 ;
- un mandrin court, apte à être inséré conjointement dans le manchon et dans une lumière interne de la sonde par l'extrémité proximale de celle-ci ;
- un outil de manchonnage comportant un logement distal apte à recevoir le mandrin court et à être enfilé conjointement sur l'extrémité proximale monodiamètre de la sonde ; cet outil comporte très avantageusement un gabarit de profondeur, apte à ajuster la profondeur de pénétration axiale du manchon sur l'extrémité proximale monodiamètre de la sonde.

On va maintenant décrire un exemple de mise en oeuvre du nécessaire de mise en place de l'invention, en référence aux dessins annexés.

La figure 1 illustre schématiquement les différentes étapes (a) à (i) de mise en place de la sonde coronaire au moyen du nécessaire de l'invention.

Les figures 2 à 9 sont des vues perspectives montrant de façon plus précise la manière dont sont associés entre eux les différents éléments intervenant dans cette procédure de mise en place.

La figure 10 montre l'extrémité proximale de la sonde et le manchon, avant mise en place, de ce dernier sur l'extrémité de sonde.

Les figures 11 et 12 montrent, respectivement en élévation et en coupe, l'extrémité proximale de la sonde pourvue de son manchon.

Les figures 13 et 14 montrent, respectivement en coupe et en perspective cavalière, un outil de mise en place du manchon sur l'extrémité proximale de la sonde.

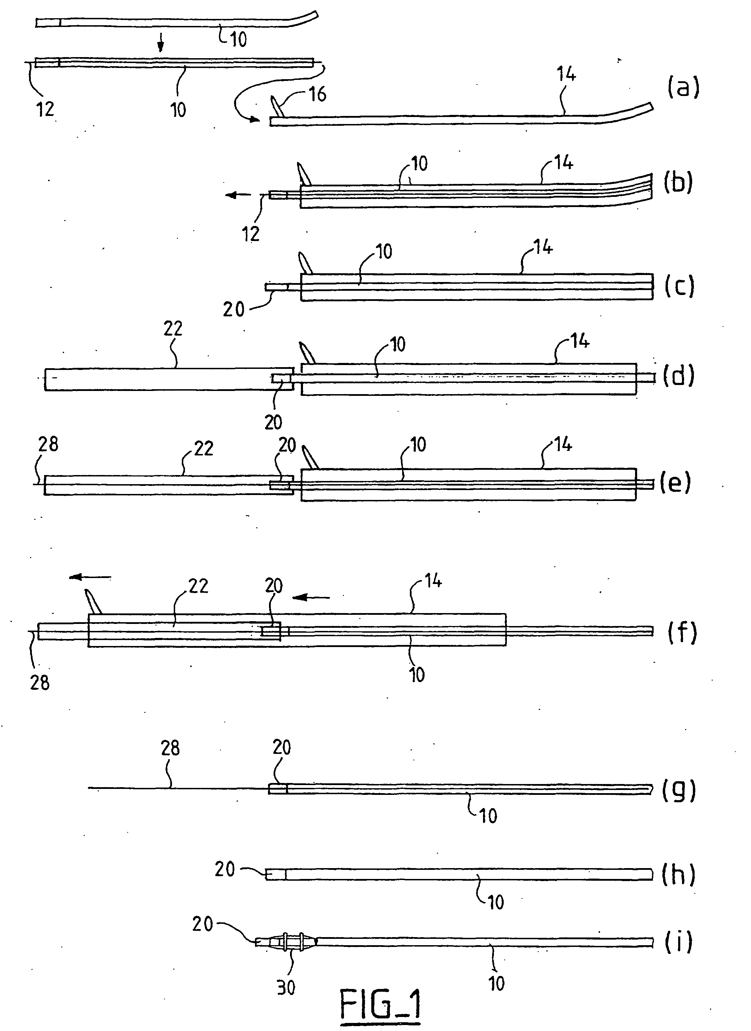

On va maintenant décrire le protocole d'utilisation du nécessaire de mise en place selon l'invention, ainsi que les divers accessoires qui le constituent, en référence aux figures 1 à 9.

Sur ce figures, la référence 10 désigne une sonde coronaire, par exemple une sonde d'un type comparable à celui décrit dans le EP-A-0 993 840 précité.

Cette sonde 10 est associée à un mandrin ou stylet 12 en matériau relativement rigide, que l'on peut faire pénétrer plus ou moins dans le corps de sonde et déformer à façon pour permettre une meilleure orientation générale de l'extrémité distale de la sonde lors de la mise en place (on pourra se référer au EP-A-0 993 840 précité pour plus de détails concernant le rôle de ce mandrin 12).

La sonde 10 pourvu de son mandrin 12 sera introduite dans le canal interne d'un cathéter-guide 14, d'un type en lui-même connu, par exemple comparable à celui décrit dans le WO-A-99/55412 précité, qui est avantageusement muni à son extrémité proximale d'une poignée de manoeuvre 16 ainsi que d'une valve hémostatique (non représentée). Ce cathéter-guide présente une rigidité en torsion suffisante pour permettre la transmission d'un mouvement de rotation d'une extrémité à l'autre, et il présente en outre une flexibilité accrue au voisinage de son extrémité distale.

Pour mettre en place le cathéter-guide dans le réseau coronaire, le chirurgien commence par placer un introducteur percutané de dimension appropriée (au moins 9 French) par la voie d'accès choisie. Il ôte le dilatateur tout en laissant en place le guide spiralé, puis introduit l'ensemble constitué par la valve hémostatique, le cathéter-guide et le dilatateur dans l'introducteur et sur le guide resté en place, conformément au mode opératoire connu de mise en place d'un tel cathéter-guide.

Une fois parvenu dans l'oreillette droite, il recule de quelques centimètres le dilatateur afin de laisser apparaître l'extrémité atraumatique du cathéter-guide. Puis, sous amplificateur de brillance, il fait pénétrer cette extrémité dans l'ostium du sinus coronaire, cette opération pouvant être facilitée par une sonde d'électrophysiologie placée dans le cathéter-guide.

Une fois le cathéter-guide mis en place, le chirurgien retire tout fil-guide ou autre appareil ayant servi au placement du cathéter-guide, ferme la valve hémostatique et purge le cathéter-guide à l'aide de la voie latérale de la valve hémostatique.

Il peut alors procéder au placement de la sonde dans le cathéter-guide ainsi mis en place (cette étape correspondant à ce qui est illustré schématiquement sur la figure 1(a)).

Pour cela, il sort la sonde de sa boîte, humidifie la surface du corps de sonde avec du sérum physiologique et enfile le mandrin 12 dans le canal interne de la sonde. Il purge à nouveau le cathéter-guide à l'aide de la voie latérale de la valve hémostatique, puis ouvre la valve et introduit dans le cathéter-guide 14 la sonde 10 rigidifiée par le mandrin droit 12. La valve hémostatique ayant été ajustée au diamètre du corps de sonde, le placement final proprement dit de celle-ci dans le réseau coronaire est alors réalisé. La configuration résultante est celle illustrée figure 1(b).

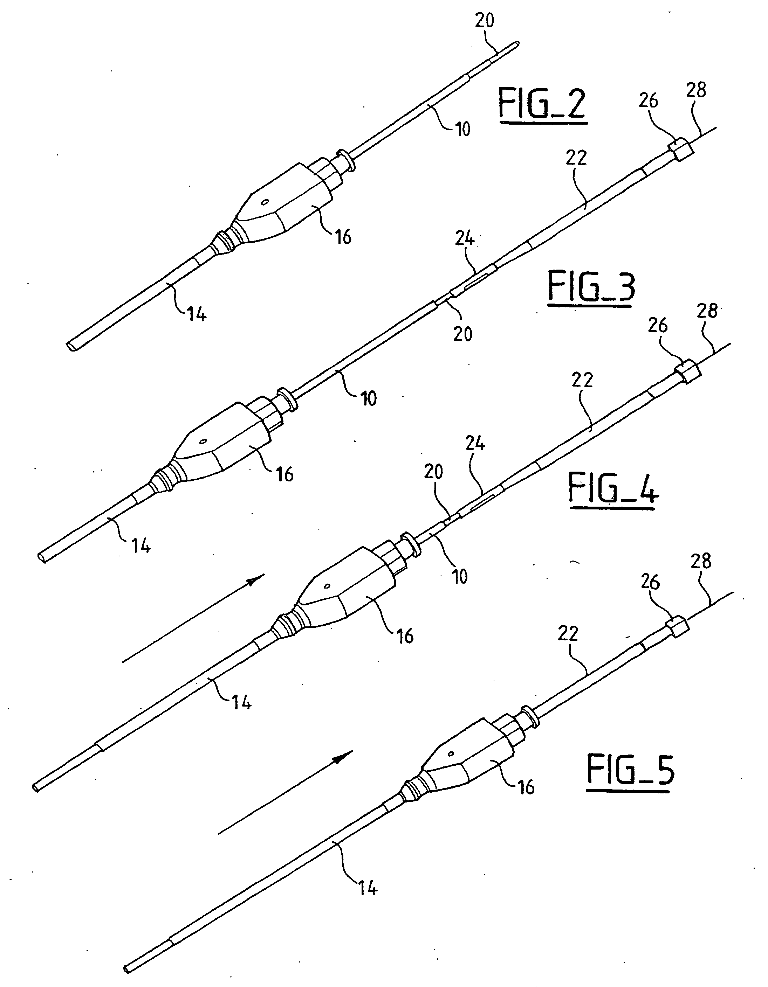

Enfin, le cathéter-guide restant en place, le chirurgien retire le mandrin 12 de la sonde 10 ; cette configuration correspond aux figures 1(c) et 2.

Le chirurgien procède alors au retrait du cathéter-guide, de la manière originale enseignée par la présente invention.

La première étape consiste à prolonger l'extrémité proximale 20 de la sonde, qui émerge du cathéter-guide, par une rallonge 22 spécifique de l'invention.

L'extrémité proximale 20 de la sonde est ici constituée d'un connecteur monodiamètre (c'est-à-dire que le diamètre de ce connecteur n'est pas supérieur à celui du corps de la sonde) destiné à être ultérieurement inséré dans la tête de connexion d'un stimulateur pour assurer la liaison électrique entre le générateur d'impulsions de ce dernier et l'électrode de stimulation, située à l'autre extrémité (l'extrémité distale) de la sonde.

La rallonge est réalisée sous forme d'un tube monodiamètre, percé, par exemple en polyuréthanne. La sonde, quant à elle, est revêtue extérieurement d'un matériau à faible coefficient de frottement (par rapport à la surface intérieure du cathéter-guide), à l'exception des extrémités, par exemple un revêtement d'un hydrogel essentiellement constitué (pour 99 %) de polyvinylpyrrolidone, avec environ 1 % d'agent d'adhésion de la couche de polyvinylpyrrolidone sur le silicone du tube.

La longueur de la rallonge 22 est légèrement supérieure à celle du cathéter-guide 14 ; son diamètre est inférieur au diamètre intérieur de la lumière du cathéter-guide 14. Elle se présente une bonne tenue mécanique (c'est-à-dire une certaine raideur qui lui assure un maintien propre lorsqu'elle est tenue seulement à l'une de ses extrémités) et ne requérant qu'un faible effort d'insertion pour sa solidarisation (ici par emboîtement) sur le connecteur monodiamètre 20 : la rallonge est par exemple pourvue d'un logement femelle 24 (figure 3) formant douille, homologue du connecteur monodiamètre 20 de la sonde 10, et par exemple fendu sur une partie de sa longueur pour permettre un ajustement avec serrage lorsque ces deux éléments ont été enfichés l'un dans l'autre.

Une fois la rallonge 22 montée, la configuration est celle illustrée figure 1(d).

Le chirurgien insère alors par l'extrémité proximale 26 (figure 3) de la rallonge 22 un mandrin 28 dont la longueur est supérieure à la somme des longueurs de la sonde 10 et de la rallonge 22. Ce mandrin long 28 permet de maintenir en position la sonde 10 et évite son délogement éventuel lors des étapes suivantes ; il est souhaitable d'enfoncer le mandrin long 28 jusqu'à dépasser le sinus coronaire d'au moins quelques centimètres, voire jusqu'à l'extrémité de la sonde.

La configuration de l'ensemble est alors celle illustrée figures 1(e) et 3.

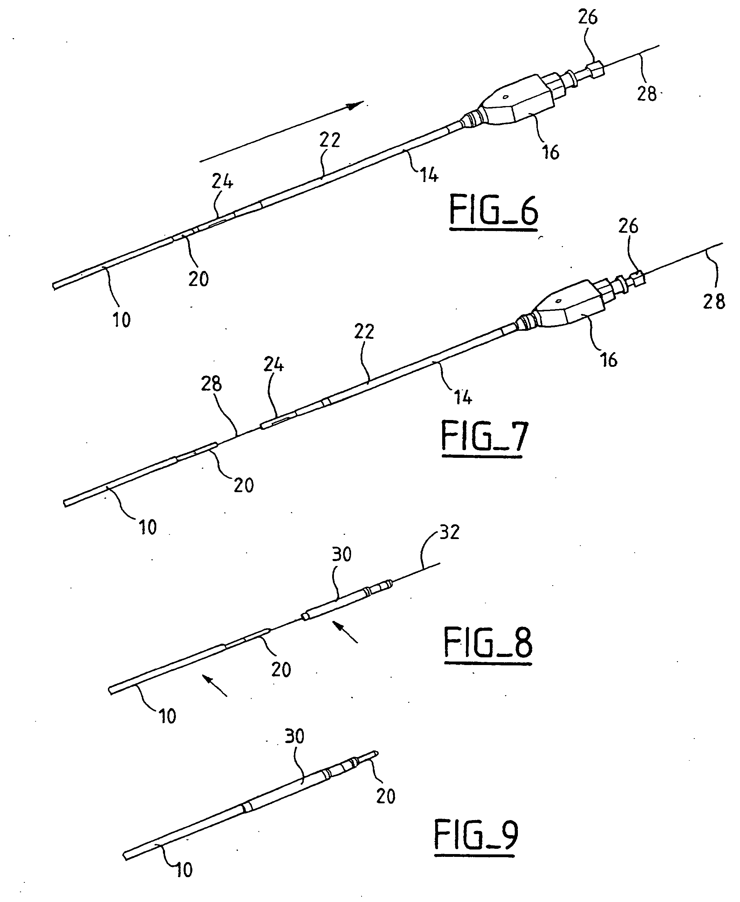

L'étape suivante consiste, sous amplificateur de brillance, à faire glisser le cathéter-guide 14 sur la sonde 10 (position intermédiaire illustrée figure 4), puis sur la rallonge 22 (position intermédiaire illustrée figures 1(f) et figure 5) jusqu'à ce que le cathéter-guide 14 soit complètement dégagé de la sonde. Les figures 6 et 7 illustrent d'autres positions intermédiaires.

Le chirurgien déconnecte alors la rallonge (configuration de la figure 1(g)) et, sous amplificateur de brillance, retire le mandrin long 28 (configuration de la figure 1(h)).

La dernière étape est une étape de finition du connecteur : en effet, l'extrémité proximale de la sonde étant un simple connecteur monodiamètre 20, il est nécessaire d'adapter sur ce dernier un manchon 30 pour transformer le connecteur monodiamètre en une fiche de connexion conforme notamment au système de connexion normalisé IS-1 de connecteur de sonde coronaire, pour aboutir à la configuration de la figure 1(i), où la sonde 10 est prête à être raccorder au stimulateur ou défibrillateur par la fiche de connexion électrique constituée du connecteur monodiamètre 20 et de son manchon 30.

À cet égard, on pourra se reporter à la norme française et européenne NF EN 50077 *"Connecteur à bas profil pour stimulateurs cardiaques implantables"*, qui définit le système de connexion normalisé dit "IS-1" permettant de garantir l'interchangeabilité des sondes et des générateurs d'impulsions produits par différents fabricants (l'invention n'est toutefois pas limitée au cas particulier des systèmes de connexion selon cette norme, ni même aux systèmes de connexion pour stimulateurs cardiaques). Plus précisément, le boîtier du dispositif est constitué d'un corps contenant les divers circuits électroniques et la pile d'alimentation du dispositif, et d'une tête de connecteur reliée mécaniquement et électriquement au corps et pourvu d'un ou plusieurs logements susceptibles de recevoir la ou les sonde(s).

Après avoir nettoyé le connecteur monodiamètre 20 et trempé l'extrémité du manchon 30 dans une fiole d'huile minérale, le chirurgien enfile dans ce manchon 30 un mandrin 32, comme illustré figure 8.

Il trempe alors l'extrémité du connecteur monodiamètre 20 dans la fiole d'huile minérale, puis insère le mandrin 32 dans le canal interne de la sonde pour rigidifier cette dernière, sur une longueur de l'ordre de 10 à 15 cm. Il fait enfin glisser le manchon 30 sur le connecteur monodiamètre 20 jusqu'à ressentir une butée franche. Le mandrin 32 est ensuite retiré et le surplus d'huile essuyé. La configuration finale obtenue est celle des figures 1(i) et 9.

La fiche de connexion ainsi réalisée peut alors, après lubrification, être insérée dans la tête de connecteur du stimulateur, où elle sera enfin maintenue en place par serrage des vis de blocage prévues sur cette tête de connecteur.

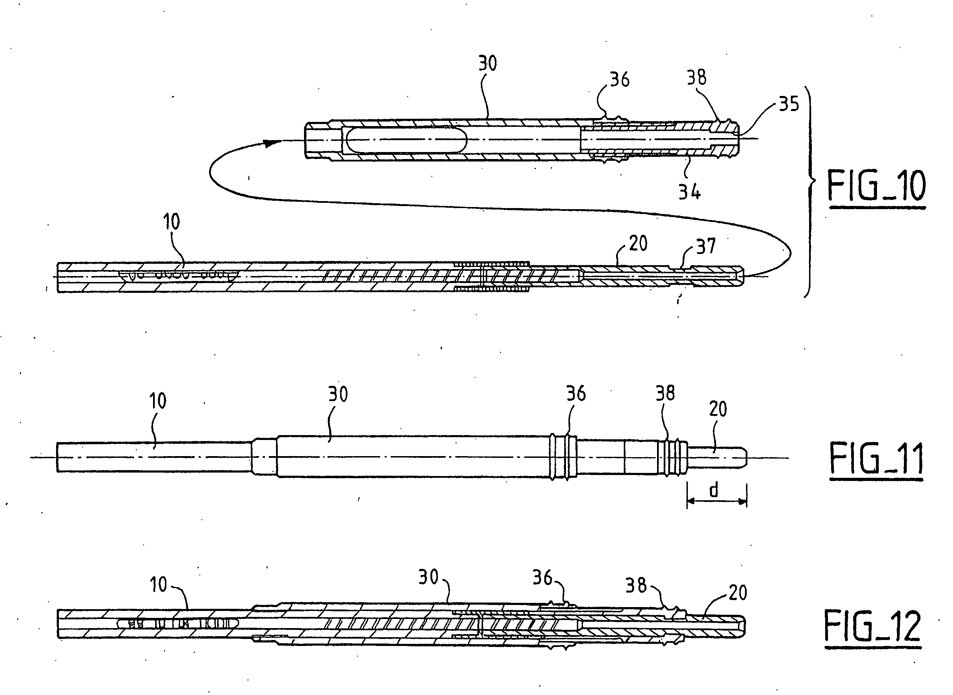

La figure 10 montre plus en détail, en coupe, les deux parties homologues du connecteur monodiamètre 20 de la sonde 10 et du manchon 30.

Le diamètre intérieur de la partie 34 du manchon 32 recevant l'extrémité de la sonde correspond à celui du connecteur monodiamètre 20. Une région de moindre diamètre 35 à l'extrémité proximale du manchon 30 coopère avec un étranglement homologue 37 du connecteur 20, de manière à produire un effet d'encliquetage lors de l'assemblage de ces deux éléments et empêcher ensuite tout déplacement longitudinal mutuel de ces deux éléments, afin de respecter les cotes du standard IS-1.

Les figures 11 et 12 montrent la fiche obtenue après manchonnage du connecteur de la sonde 10, respectivement en élévation et en coupe.

Comme on peut le voir, l'extrémité libre du connecteur monodiamètre 20 dépasse la partie 34 dans laquelle il a été emboîté, et c'est sur cette partie émergeante que sera réalisée la liaison électrique avec la tête de connecteur du boîtier du stimulateur. Le manchon 32 porte des reliefs périphériques tels que 36, 38 permettant d'obtenir une étanchéité entre la fiche et le logement de la tête du connecteur recevant cette fiche, en conformité avec la norme IS-1 précitée.

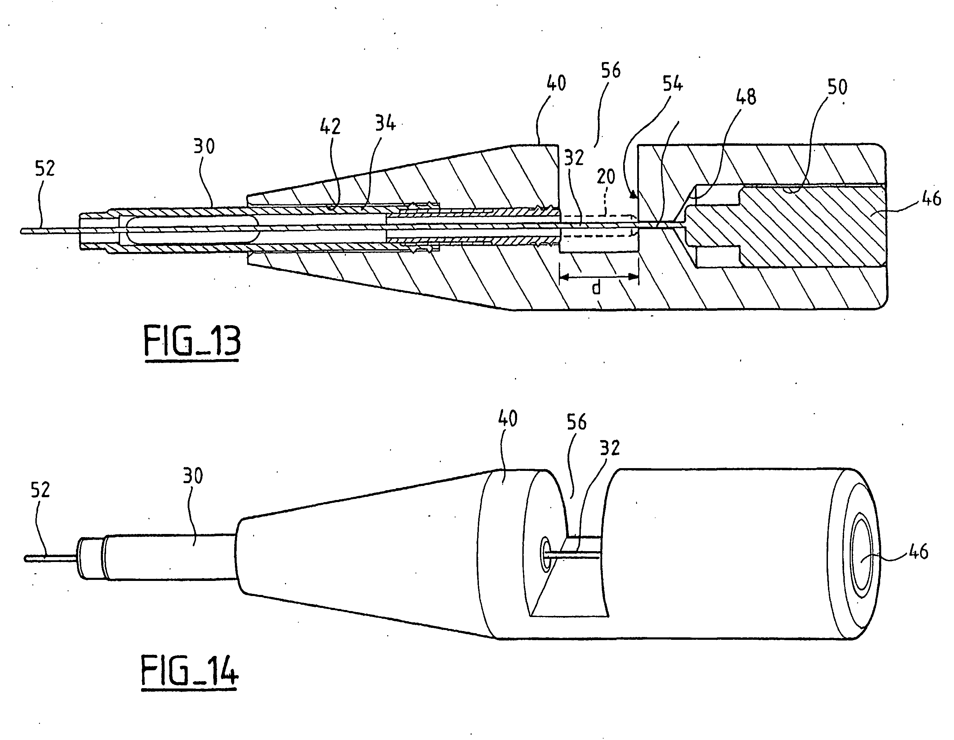

Le manchonnage du connecteur monodiamètre 20 de la sonde 10 peut être avantageusement réalisé au moyen d'un outil *ad hoc* tel que celui illustré figures 13 et 14.

Cet outil 40 comporte un logement distal 42 apte à recevoir la partie 34 du manchon 30 devant venir entourer le connecteur monodiamètre. Très avantageusement, il est pré-équipé en usine du manchon 30 pour une utilisation directe par le praticien.

L'outil 40 comporte également en partie proximale un orifice 44 dans lequel peut être inséré le mandrin 32 après mise en place du manchon 30 (lorsque l'outil 40 est pré-équipé du manchon 30, le mandrin 32 est également mis en place en usine dans l'outil 40). Côté proximal, ce mandrin est terminé par une poignée 46 venant en butée, lorsque le mandrin est enfoncé sur la longueur voulue, contre le fond 48 d'une cavité proximale 50 de l'outil 40. Dans cette position, le mandrin 32 traverse entièrement le manchon 30, duquel il vient émerger côté distal en 52, sur une longueur de l'ordre d'une dizaine de centimètres.

Le connecteur monodiamètre de la sonde est alors enfilé sur cette partie émergeante 52 du mandrin, et il vient pénétrer progressivement à l'intérieure du manchon 30 sur toute la longueur de celui-ci, puis au-delà, de sorte que l'extrémité du connecteur monodiamètre 20 émerge dans la position représentée en tiretés sur la figure 13, jusqu'à venir en butée contre une paroi 54 d'une cavité 56 ménagée en partie centrale de l'outil 40.

L'outil 40 et le mandrin 32 sont ensuite aisément retirés par traction, une fois la pièce 30 mise en place.

L'outil 40 sert ainsi de gabarit pour permettre le manchonnage du connecteur monodiamètre 20 sur une longueur permettant d'ajuster très précisément la dimension d (figures 11 et 13) de la partie émergeante.

## Revendications

1. Un nécessaire de mise en place d'une sonde de stimulation d'une cavité cardiaque implantable dans le réseau coronarien, cette sonde étant une sonde de type monodiamètre à son extrémité proximale, **caractérisé en ce qu'**il comprend, en combinaison :
a) un cathéter-guide (14), comportant une lumière interne ouverte à ses deux extrémités et apte à recevoir la sonde (10) sur toute sa longueur, et
b) une rallonge (22) comportant à son extrémité distale des moyens (24) de solidarisation à l'extrémité proximale monodiamètre (20) de la sonde (10),
et **en ce que** :
- le diamètre extérieur des moyens de solidarisation est le même que celui du reste de la rallonge,
- la surface extérieure de la rallonge est une surface à faible coefficient de frottement par rapport à la surface intérieure de la lumière du cathéter-guide, et
- le diamètre extérieur dé cette rallonge est inférieur au diamètre intérieur de la lumière du cathéter-guide,
de manière à permettre un glissement axial du cathéter-guide sur toute la longueur de la rallonge, sans déplacement axial concomitant de la sonde.

2. Le nécessaire de la revendication 1, comprenant en outre :
c) un mandrin long (28), apte à être inséré dans une lumière interne de la rallonge par une extrémité proximale (26) de celle-ci, la longueur de ce mandrin étant supérieure à la somme des longueurs de la sonde et de la rallonge.

3. Le nécessaire de la revendication 1, comprenant en outre :
d) un manchon (30) apte à être emboîté sur l'extrémité proximale monodiamètre (20) de la sonde (10), de manière à former une fiche de connexion électrique raccordable à une tête de connecteur d'un stimulateur ou défibrillateur.

4. Le nécessaire de la revendication 3, dans lequel les dimensions et la forme du manchon (30) sont choisis en conformité avec la norme du système de connexion IS-1.

5. Le nécessaire de la revendication 3, comprenant en outre :
e) un mandrin court (32), apte à être inséré conjointement dans le manchon (30) et dans une lumière interne de la sonde par l'extrémité proximale (20) de celle-ci.

6. Le nécessaire de la revendication 5, comprenant en outre :
f) un outil de manchonnage (40) comportant un logement distal (42) apte à recevoir le mandrin court (32) et à être enfilé conjointement sur l'extrémité proximale monodiamètre (20) de la sonde.

7. Le nécessaire de la revendication 6, dans lequel l'outil de manchonnage (40) comporte un gabarit de profondeur (54), apte à ajuster la profondeur (d) de pénétration axiale du manchon (30) sur l'extrémité proximale monodiamètre (20) de la sonde.

8. Le nécessaire de l'une des revendications 1, 2 ou 3, incluant en outre ladite sonde de stimulation.

## Patentansprüche

1. Vorrichtung zum Einsetzen einer in den Koronargefäßen implantierbaren Sonde zur Stimulation einer Herzkammer, wobei diese Sonde eine Sonde vom Typ Monodurchmesser an ihrem proximalen Ende ist, **dadurch gekennzeichnet, dass** sie in Kombination umfasst:
a) eine Katheterführung (14), aufweisend einen inneren Kanal, der an seinen zwei Enden offen ist und in der Lage ist, die Sonde (10) auf ihrer ganzen Länge zu empfangen, und
b) eine Verlängerung (22), aufweisend an ihrem distalen Ende Mittel (24) zur Befestigung an das proximale Monodurchmesserende (20) der Sonde (10),
und **dadurch**, dass:
- der äußere Durchmesser der Mittel zur Befestigung der gleiche ist wie derjenige des Rests der Verlängerung,
- die äußere Oberfläche der Verlängerung eine Oberfläche mit schwachem Reibungskoeffizienten ist im Vergleich zur inneren Oberfläche des Kanals der Katheterführung, und
- der äußere Durchmesser dieser Verlängerung kleiner ist als der innere Durchmesser des Kanals der Katheterführung,
um ein axiales Gleiten der Katheterführung auf der ganzen Länge der Verlängerung ohne begleitende axiale Verschiebung der Sonde zu ermöglichen.

2. Vorrichtung gemäß Anspruch 1, weiterhin aufweisend:
c) einen langen Dorn (28), dazu geeignet, in einen internen Kanal der Verlängerung durch ein proximales Ende (26) von dieser eingeführt zu werden, wobei die Länge dieses Doms größer ist als die Summe der Längen der Sonde und der Verlängerung.

3. Vorrichtung gemäß Anspruch 1, weiterhin aufweisend:
d) eine Muffe (30), dazu geeignet, auf dem proximalen Monodurchmesserende (20) der Sonde (10) eingepasst zu werden, um einen elektrischen Verbindungsstecker zu bilden, der mit einem Steckerkopf eines Stimulators oder Defibrillators verbunden werden kann.

4. Vorrichtung gemäß Anspruch 3, in der die Abmessungen und die Form der Muffe (30) in Übereinstimmung mit der Norm des Steckersystems IS-1 gewählt sind.

5. Vorrichtung gemäß Anspruch 3, weiterhin aufweisend:
e) einen kurzen Dorn (32), der dazu geeignet ist, zusammen in die Muffe (30) und in einen internen Kanal der Sonde durch deren proximales Ende (20) eingeführt zu werden.

6. Vorrichtung gemäß Anspruch 5, weiterhin aufweisend:
f) ein Werkzeug zum Muffen (40), aufweisend eine distale Unterbringung (42), die dazu geeignet ist, den kurzen Dorn (32) zu empfangen und gemeinsam auf dem proximalen Monodurchmesserende (20) der Sonde aufgefädelt zu werden.

7. Vorrichtung gemäß Anspruch 6, in der das Werkzeug zum Muffen (40) eine Tiefenschablone (54) aufweist, die dazu geeignet ist, die Tiefe (d) der axialen Penetration des Doms (30) am proximalen Monodurchmesserende (20) der Sonde einzustellen.

8. Vorrichtung gemäß einem der Ansprüche 1, 2 oder 3, weiterhin umfassend die Stimulationssonde.

## Claims

1. A kit for placing a cardiac cavity stimulation lead in the coronary network, said lead being a monodiameter type lead at its proximal end, **characterised by** comprising, in combination:
a) a guiding catheter (14) comprising an internal lumen open at its two ends and adapted to receive the lead (10) along its length, and
b) an extension (22) comprising at its distal end means (24) that mounts to the monodiameter proximal end (20) of the lead (10),
and in that:
- the external diameter of the mounting means is the same as the one of the reminder of the extension,
- the external surface of the extension is a low friction coefficient surface as compared to the interior surface of the lumen of the guiding catheter, and
- the external diameter of said extension is smaller than the interior diameter of the lumen of the guiding catheter,
whereby an axial slip of the guiding catheter along the entire length of the extension is permitted without a concomitant axial displacement of the lead.

2. The kit of claim 1, further comprising:
c) a long stylet (28) adapted to be inserted in an internal lumen of the extension through a proximal end (26) of the latter, the length of said stylet being greater than the sum of the lengths of the lead and of the extension.

3. The kit of claim 1, further comprising:
d) a sleeve (30) adapted to be fitted on the monodiameter proximal end (20) of the lead (10), so as to form an electrical connection plug connectable to a connector head of a pacemaker or defibrillator.

4. The kit of claim 3, wherein the dimensions and the shape of the sleeve (30) are chosen in conformity with the IS-1 system connection standard.

5. The kit of claim 3, further comprising:
e) a short stylet (32) adapted to be jointly inserted in the sleeve (30) and in an internal lumen of the lead through the proximal end of the latter.

6. The kit of claim 5, further comprising:
f) a sleeving tool (40) comprising a distal housing (42) adapted to receive the short stylet (32) and to be jointly threaded on the monodiameter proximal end (20) of the lead.

7. The kit of claim 6, wherein the sleeving tool (40) includes a depth gauge (54) adapted to adjust the axial penetration depth (d) of the sleeve (30) relative to the monodiameter proximal end (20) of the lead.

8. The kit of any of claims 1, 2, or 3, further including said stimulation lead.
